# EUROPÄISCHE PATENTSCHRIFT

(11) **EP 1 153 577 B1**
(45) Veröffentlichungstag und Bekanntmachung des Hinweises auf die Patenterteilung: **26.09.2007**
(21) Anmeldenummer: 01810366.3
(22) Anmeldetag: 12.04.2001
(51) Int. Cl.: A61B 17/70, A61B 17/80

(54) **Verbindungsanordnung umfassend eine Knochenschraube und eine Knochenplatte**
Fixation comprising a bone screw and a bone plate
Fixation contenant une vis et une plaque d'ostéosynthèse

(30) Priorität: 12.05.2000 EP 00810406
(43) Veröffentlichungstag der Anmeldung: 14.11.2001
(73) Patentinhaber: Zimmer GmbH, 8404 Winterthur (CH)
(72) Erfinder: Donno, Cosimo, 8400 Winterhur (CH); Casutt, Simon, 9200 Gossau (CH)
(74) Vertreter: Manitz, Finsterwald & Partner GbR

(56) Entgegenhaltungen:
- EP-A- 0 988 833
- WO-A-00/03653
- DE-A- 19 542 116
- FR-A- 2 778 088
- US-A- 6 022 350

## Beschreibung

Die Erfindung handelt von einer Verbindung von einer Knochenschraube zu einer Knochenplatte gemäß dem Oberbegriff des Anspruchs 1.

Ein Problem dieser Anwendungen besteht darin, dass eine einmal gesetzte Knochenschraube mit ihrem Kopf eine biegesteife Verbindung zu dem Implantat bilden sollte, die unabhängig von den Verankerungskräften zwischen Knochenschraube und Knochen ist. So zeigt die EP-A-0 988 833 eine biegesteife Verbindung zwischen einer Knochenschraube mit Kugelkopf und einer Knochenplatte. Ein weiteres Problem für eine derartige Verbindung besteht darin, dass sie eine Bauhöhe beansprucht, die sich nicht ohne weiteres reduzieren lässt. In Anwendungsbereichen wie zum Beispiel bei Halswirbeln wären dünne Knochenplatten, die auch im Bereich der Verbindung nur unwesentlich dicker sind, von Vorteil.

DE 194 42 116 A1 beschreibt eine Verbindung mit einen Schwenkbaren Knochenschraube, die mit einem zentralen Kugelansatz in eine entsprechende Kugelpfanne eines Schraubeinsatzes eintaucht.

Aufgabe der Erfindung ist es, eine Verbindungsanordnung der eingangs genannten Art zu schaffen, die bei möglichst guter Fixierbarkeit und möglichst großem Schwenkbereich der Knochenschraube eine möglichst geringe Bauhöhe beansprucht.

Diese Aufgabe wird durch die Merkmale des Anspruchs 1 gelöst, dass die Höhe der Knochenplatte im Bereich der Knochenschraube kleiner oder gleich dem Durchmesser des Schaftes ist, dass der Schraubenkopf in eine Ausnehmung der Sicherungsschraube eintaucht, und dass die Sicherungsschraube mit der Oberseite der Knochenplatte abschliesst.

Eine derartige Ausführung der im Folgenden auch einfach kurz als Verbindung bezeichneten Verbindungsanordung, die Bauhöhen gestattet, welche höchstens dem Schaftdurchmesser der Knochenschraube entsprechen, und die trotzdem eine starre, biegesteife Verbindung ergeben, verursacht weder unnötig dicke Knochenplatten noch zusätzlich vorstehende Kanten oder Flächen, die dem Träger des Implantats Druckstellen zwischen Implantat und Aussenhaut erzeugen.

Vorteilhafte Weiterbildungen der Erfindung ergeben sich aus den abhängigen Ansprüchen 2 bis 9. Die Konstruktion gestattet es trotz der geringen . Bauhöhe der Verbindung für die Gewindelänge der Sicherungsschraube deutlich mehr als die Hälfte der Bauhöhe vorzusehen. Mit der Ausführung des Kopfes der Knochenschraube als kugelförmige Pfanne und passenden Vor- und Rücksprüngen an der Sicherungsschraube kann die kugelförmige Pfanne in Aussparungen geschwenkt werden, die hinter dem Gewinde der Sicherungsschraube liegen. Passende Aussparungen sind beispielsweise möglich, wenn der Gewindedurchmesser der Sicherungsschraube doppelt so gross wie der Schaftdurchmesser der Knochenschraube ist. Dies trifft auf eine schwenkbare, mit einer kugelförmigen Pfanne ausgeführte Knochenschraube zu. Wenn die letztere mit ihrer kugelförmigen Pfanne Aussenflächen und Innenflächen mit gleichem Kugelmittelpunkt aufweist, lassen sich Schwenkungen aus einer Mittelstellung um Winkel α₁, α₂ durchführen, die bis gegen 20° betragen können, um anschliessend die Knochenschraube mit der Sicherungsschraube zu fixieren. Diese Art der Ausführung hat den Vorteil, dass die Fixierung der Knochenplatte weitgehend unabhängig von der Winkellage der Knochenschrauben erfolgen kann. Dabei ist die Knochenplatte nicht nur auf die Verbindung von Knochenbruchstücken beschränkt, sondern kann auch eine Stützkonstruktion zwischen zwei Wirbeln sein.

Bei einer Verwendung einer Knochenplatte zwischen zwei Wirbeln, kann der überbrückende Teil starr oder auch als Biegefeder ausgebildet sein, um Kräfte von einem Wirbel auf den anderen Wirbel zu übertragen.

Es besteht auch die Möglichkeit an zwei Wirbeln jeweils eine Knochenplatte als Verankerungskörper in Form eines Jochs mit mehreren Knochenschrauben auszuführen und als eigentliche Stützkonstruktion ein elastisches Band mit einem elastischen Druckkörper zu verwenden, wie es in der EP-B-0 669 109 gezeigt ist.

Als Knochenschrauben für die Verankerung sind Knochenschrauben mit einem Schaftdurchmesser von 2 bis 10 mm geeignet.

Im folgenden wird die Erfindung anhand von Ausführungsbeispielen beschrieben. Es zeigen:
- Fig. 1: Schematisch zwei benachbarte Wirbel, an denen jeweils eine als Joch ausgeführte erfindungsgemässe Knochenplatte mit Knochenschrauben verankert ist, und als Verbindung der beiden Knochenplatten ein elastisches Band mit einem elastischen Druckkörper;
- Fig. 2: schematisch eine Draufsicht der Figur 1;
- Fig. 3: schematisch eine Seitenansicht der Figur 1;
- Fig. 4: schematisch und vergrössert einen Längsschnitt der Figur 1;

- Fig.5: schematisch und allgemein einen stark vergrösserten Ausschnitt der Erfindung, bei welcher der Kopf der Knochenschraube schwenkbar ist;
- Fig.6: schematisch eine Anwendung der Erfindung mit einer Knochenplatte für die Osteosynethese;
- Fig. 7: schematisch eine Ausführung zwischen zwei benachbarten Wirbeln, bei der die Knochenplatte als Biegefeder die beiden Wirbel verbindet; und
- Fig. 8: schematisch eine Ausführung wie in Figur 7 mit einer anderen Form der Biegefeder.

In den Figuren ist die Verbindung einer Knochenschraube 12 zu einer Knochenplatte 7 gezeigt. Der Kopf 13 der Knochenschraube 12 liegt mit einer ringförmigen Aussenfläche 10 auf einer Gegenfläche auf und ist mit einer in der Richtung zur Gegenfläche einschraubbaren Sicherungsschraube 15 fixierbar. Dabei ist die Höhe 16 der Knochenplatte 7 so gewählt, dass sie kleiner oder gleich dem Schaftdurchmesser 19 der Knochenschraube 12 ist, während der Schraubenkopf 13 in eine Ausnehmung 20 der Sicherungsschraube 15 eintaucht und die Oberseite der Sicherungsschraube 15 mit der Oberseite der Knochenplatte 7 abschliesst.

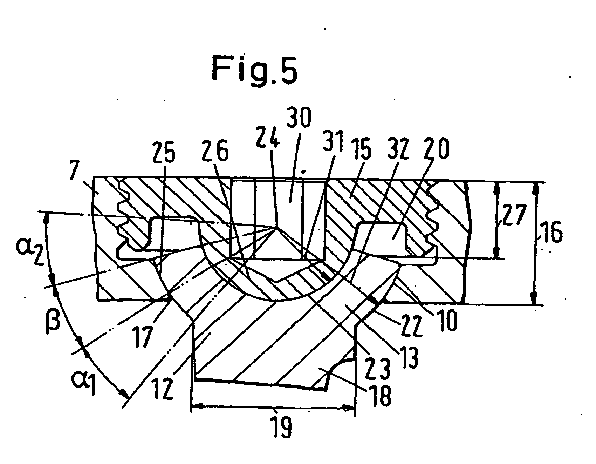

Im allgemeinen Beispiel der Figur 5 ist der Kopf 13 einer Knochenschraube 12 als kugelförmige Pfanne mit einer kugelförmigen Aussenfläche 22 und einer kugelförmigen Innenfläche 23 ausgeführt, welche einen gemeinsamen Mittelpunkt 24 besitzen. Die gleichzeitig ringförmige 10 Aussenfläche 22 sitzt auf einer Gegenfläche 25 auf. Die Ringfläche 10 endet mit dem Schaftdurchmesser 19, welcher kleiner ist als der Durchmesser einer Durchtrittsöffnung 17, welche durch die Gegenfläche 25 gebildet wird. Bezogen auf den Mittelpunkt 24 und eine Mittelstellung der Knochenschraube 12 kann die Knochenschraube in jeder Richtung um einen Schwenkwinkel α₁ geschwenkt werden. In der Sicherungsschraube 15 ist eine Ausnehmung 20 eingearbeitet, die ein Schwenken der kugelförmigen Pfanne um einen Winkel α₂ erlaubt, der etwa gleich gross wie der Winkel α₁ ist. Die Gegenfläche 25 bildet einen Winkel β zum Mittelpunkt 24, wobei der Winkel β grösser als der Schwenkwinkel α₁ ist, damit Aussenfläche 22 und Gegenfläche 25 in jeder möglichen Winkellage Kontakt haben. Eine Sicherungsschraube 15 ragt mit ihrem Kern 26 bis in den Grund der Innenfläche 23 und schafft gleichzeitig Raum für einen Innensechskant 30. Der Radius 31 der Innenfläche 23 und der Radius 32 der Aussenfläche 22 bestimmen die Durchmesser der ringförmigen Ausnehmung 20. Der Gewindedurchmesser der Sicherungsschraube 15 ist doppelt so gross wie der Schaftdurchmesser 19 gewählt, um genügend Material zwischen Ausnehmung 20 und dem Gewinde zu haben. Die Gewindelänge 27 beträgt 60 % der Höhe 16 der Knochenplatte 7. Die Höhe 16 beträgt 85 % des Schaftdurchmessers 19 der Knochenschraube 12. In den Schaft 18 der Knochenschraube 12 ist ein Gewinde eingeschnitten.

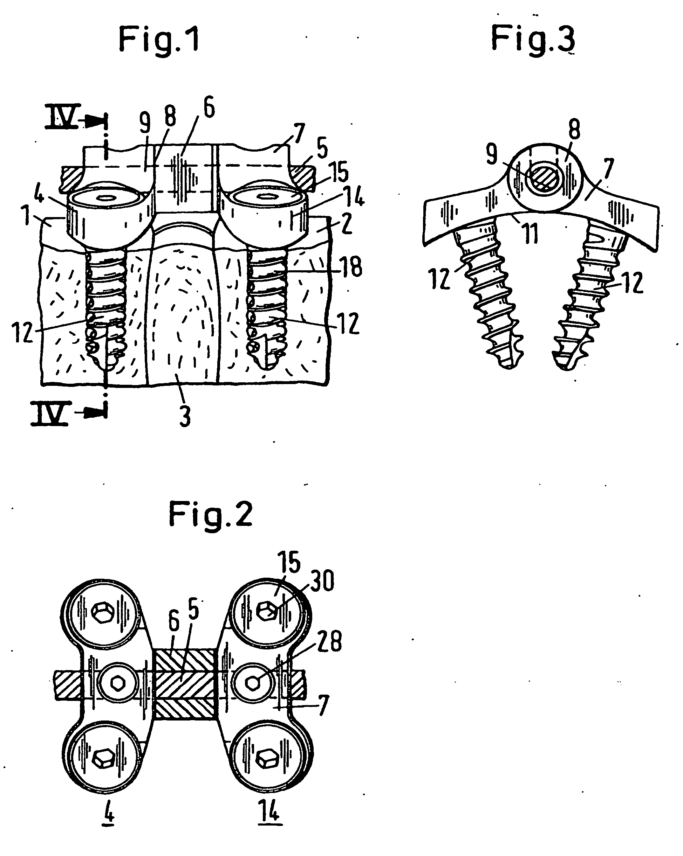

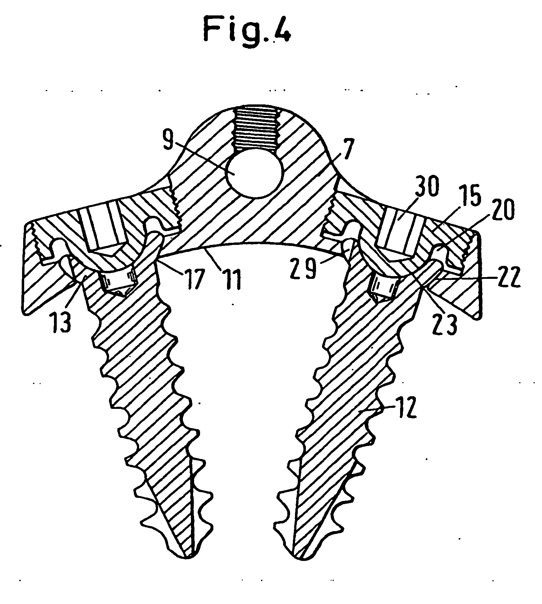

Im Ausführungsbeispiel der Figuren 1, 2, 3 und 4 bildet die Knochenplatte jeweils ein Joch mit beidseitigen Durchtrittsöffnungen 17, in welchen der Kopf 13 einer Knochenschraube 12 anliegt (Figur 4). Eine erste Knochenschraube 12 wird mit ihrem Schaft durch die Durchtrittsöffnung 17 gesteckt und mit einem Schraubwerkzeug (nicht gezeigt) an Aussparungen 29 aufgenommen und in eine vorbereitete Knochenbohrung eingedreht. Eine zweite Knochenschraube wird auf der Gegenseite ebenfalls eingedreht bis die Anlageseite 11 des Jochs am Knochen anliegt. Anschliessend werden Sicherungsschrauben 15 an ihrem Innensechskant 30 mit einem Schlüssel angezogen, um in der durch den Knochen bestimmten Winkellage der Knochenschrauben 12 eine starre Verbindung zwischen Knochenschraube und Joch zu erzeugen. Im vorliegenden Beispiel bilden zwei auf benachbarten Wirbeln 1, 2 befestigte Knochenplatte 7 die Basis für eine elastische Stützkonstruktion, bei der ein elastischer Druckkörper 6 und ein elastisches Zugband 5 gegeneinander verspannt sind, um begrenzte Bewegungen zwischen den beiden Wirbeln 1, 2 zuzulassen und eine dazwischen liegende Bandscheibe 3 zu entlasten. Das Zugband 5 ist jeweils mit einer Madenschraube 28 in einer Durchgangsbohrung 9 des Jochs befestigt. Der Druckkörper 6 liegt jeweils an einer ringförmigen Anlagefläche 8 des Jochs auf.

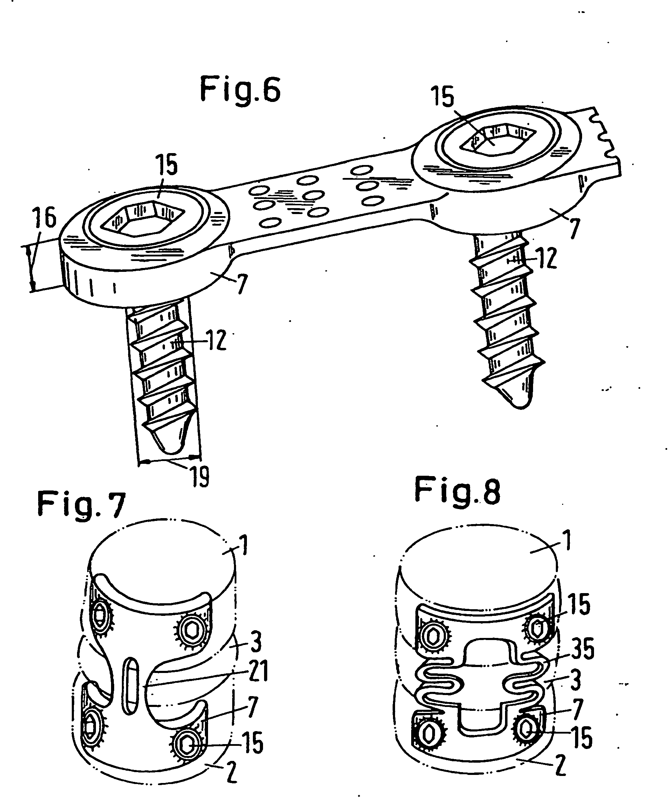

Ähnliche Anwendungen zeigen die Ausführungsbeispiele von Figur 7 und Figur 8, bei denen die Knochenplatten und die Stützkonstruktion einteilig ausgeführt sind. Eine Knochenplatte 7 ist jeweils mit zwei Knochenschrauben und über Sicherungsschrauben 15 mit zwei benachbarten Wirbeln 1, 2 verbunden und besitzt eine Brücke 21, welche die Distanz zwischen den beiden Wirbeln überbrückt. Die Brücke 21 kann mehr oder weniger starr sein. Im Beispiel der Figur 8 ist sie als doppelte, mäanderförmige Biegefeder 35 ausgeführt. Derartige platzsparende Ausführungen sind im Bereich der Halswirbel von Vorteil.

Eine allgemeine Ausführung für die Osteosynthese ist mit dem Beispiel von Figur 6 gezeigt. Längs einer Knochenplatte 7 können Knochenschrauben 12 in verschiedene Knochenstücke (nicht gezeigt) eingedreht und mit Sicherungsschrauben 15 zur Knochenplatte 7 starr verbunden werden. Im vorliegenden Fall ist die Knochenplatte zwischen den Knochenschrauben 12 geschwächt, um intraoperativ durch Verbiegen eine Anpassung an den Knochenverlauf zu gestatten. Als Vorteil wirkt sich wiederum die geringe Höhe 16 der Knochenplatte 7 aus, die kleiner ist als der Schaftdurchmesser 19 der Knochenschrauben.

Grundsätzlich lässt sich die Erfindung für alle Schaftdurchmesser von Knochenschrauben anwenden.

## Patentansprüche

1. Verbindungsanordnung umfassend eine Knochenplatte (7) und eine Knochenschraube (12), deren Kopf (13) mit einer ringförmigen Aussenfläche (10) auf einer Gegenfläche (25) der Knochenplatte (7) aufliegt und mit einer in der Knochenplatte (7) in der Richtung zur Gegenfläche (25) einschraubbaren Sicherungsschraube (15) fixierbar ist, wobei die Knochenplatte (7) eine Durchtrittsöffnung (17) für einen Schaft (18) der Knochenschraube (12) aufweist und wobei die Sicherungsschraube (15) mit der Oberseite der Knochenplatte (7) abschliesst, **dadurch gekennzeichnet, dass** die Höhe (16) der Knochenplatte im Bereich der Knochenschraube (12) kleiner oder gleich dem Durchmesser (19) des Schaftes (18) ist dass der Kopf (13) der Knochenschraube (12) als kugelförmige Pfanne ausgeführt ist, deren Aussenfläche (22) und Innenfläche (23) einen gleichen Mittelpunkt (24) aufweisen, dass die Sicherungsschraube (15) mit einem passenden Kern (26) in die Pfanne eintaucht, so dass die Knochenschraube (12) im noch nicht fixierten Zustand schwenkbar ist und, dass der Schraubenkopf (13) zumindest in einigen Schwenkstellungen der Knochenschraube (12) in eine Ausnehmung (20) der Sicherungsschraube (15) eintaucht.

2. Verbindungsanordnung nach Anspruch 1, **dadurch gekennzeichnet, dass** die Sicherungsschraube (15) eine in die Knochenplatte (7) eintauchende Gewindelänge (27) aufweist, welche mehr als die Hälfte der Höhe (16) der Knochenplatte (7) im Bereich der Knochenschraube (12) beiträgt.

3. Verbindungsanordnung nach Anspruch 1 order 2, **dadurch gekennzeichnet, dass** die Ausnehmung (20) der Sicherungsschraube (15) so bemessen ist, dass der Kopf (13) der Knochenschraube (12) in unterschiedlicher Winkellage zur Achse der Sicherungsschraube (15) fixierbar ist.

4. Verbindungsanordnung nach Anspruch 3, **dadurch gekennzeichnet, dass** bezogen auf eine Mittelstellung der Knochenschraube (12) in der Richtung der Achse der Sicherungsschraube (15) der Kopf (13) an seiner Aussenfläche (22) eine Fixierstellung mit einer Winkelauslenkung α₁ zulässt, und dass die Ausnehmung (20) der Sicherungsschraube (15) eine ähnlich grosse Winkelauslenkung α₂ für den Schraubenkopf (13) zulässt, und dass ein durch die Gegenfläche (10, 25) zum Mittelpunkt beanspruchter Winkel β grösser als die jeweiligen Winkel α₁, α₂ ist.

5. Verbindungsanordnung nach Anspruch 4, **dadurch gekennzeichnet, dass** die Winkel α₁, α₂ jeweils einem Winkel von 3° bis 20° entsprechen.

6. Verbindungsanordnung nach einem der Ansprüche 1 bis 5, mit einer Knochenplatte (7), die als ein Joch ausgebildet ist, welches als Verankerungskörper (4, 14) für eine Stützkonstruktion an einem Wirbel (1, 2) verwendbar ist.

7. Verbindungsanordnung nach einem der Ansprüche 1 bis 5, mit einer Knochenplatte (7), die als Brücke (21) zwischen zwei Wirbeln (1, 2) verwendbar ist.

8. Verbindungsanordnung nach Anspruch 7 mit einer Brücke (21), die den Abstand zwischen zwei Wirbeln (1, 2) überbrückt und als Biegefeder (35) ausgebildet ist.

9. Verbindungsanordnung nach einem der Ansprüche 1 bis 8, **dadurch gekennzeichnet, dass** der Schaftdurchmesser (19) der Knochenschraube (12) zwischen 2 und 10 mm beträgt.

## Claims

1. A connection arrangement comprising a bone plate (7) and a bone screw (12), the head (13) of which has a ring-shaped outer surface (10) lying on an opposing surface (25) of the bone plate (7) and can be fixed with a securing screw (15) which can be screwed into the bone plate (7) in the direction towards the opposing surface (25), wherein the bone plate (7) has a passage opening (17) for a shaft (18) of the bone screw (12), and wherein the securing screw (15) terminates flush with the upper side of the bone plate (7), **characterized in that** the height (16) of the bone plate in the region of the bone screw (12) is less than or equal to the diameter (19) of the shaft (18); **in that** the head (13) of the bone screw (12) is designed as a spherical socket, the outer surface (22) and the inner surface (23) of which have a common center (24); **in that** the securing screw (15) has a suitable core (26) which slips into the socket; and **in that** the screw head (13) slips in at least some pivot positions of the bone screw (12) into a cut-out (20) of the securing screw (15).

2. A connection arrangement in accordance with claim 1, **characterized in that** the securing screw (15) has a threaded length (27) which slips into the bone plate (7) and which amounts to more than half the height (16) of the bone plate (7) in the region of the bone screw (12).

3. A connection arrangement in accordance with claim 1 or claim 2, **characterized in that** the cut-out (20) of the securing screw (15) is dimensioned such that the head (13) of the bone screw (12) can be fixed at different angular positions with respect to the axis of the securing screw (15).

4. A connection arrangement in accordance with claim 3, **characterized in that**, in relation to a middle position of the bone screw (12) in the direction of the axis of the securing screw (15), the head (13) permits a fixing position with an angular deflection α₁ at its outer surface (22); and **in that** the cut-out (20) of the securing screw (15) permits an angular deflection α₂ of similar magnitude for the screw head (13); and **in that** an angle β with respect to the center which is taken up by the opposing surface (10, 25) is greater than the respective angles α₁, α₂.

5. A connection arrangement in accordance claim 4, **characterized in that** the angles α₁, α₂ correspond in each case to an angle from 3° to 20°.

6. A connection arrangement in accordance with any one of the claims 1 to 5, comprising a bone plate (7) which is formed as a yoke which can be used as an anchoring body (4, 14) for a support construction at a vertebra (1, 2).

7. A connection arrangement in accordance with any one of the claims 1 to 5, comprising a bone plate (7) which can be used as a bridge (21) between two vertebrae (1, 2).

8. A connection arrangement in accordance with claim 7, comprising a bridge (21) which bridges the distance between two vertebrae (1, 2) and is made as a bending spring (35).

9. A connection arrangement in accordance with any one of the claims 1 to 8, **characterized in that** the shaft diameter (19) of the bone screw (12) amounts to between 2 and 10 mm.

## Revendications

1. Agencement de liaison comprenant une plaque à os (7) et une vis à os (12), dont la tête (13) s'applique par une surface extérieure annulaire (10) sur une surface antagoniste (25) de la plaque à os (7) et peut être fixée avec une vis de blocage (15) susceptible d'être vissée dans la direction de la surface antagoniste (25) dans la plaque à os (7), dans lequel la plaque à os (7) présente une ouverture traversante (17) pour une tige (18) de la vis à os (12), et dans lequel la vis de blocage (15) se termine avec la face supérieure de la plaque à os (7) ;
**caractérisé en ce que** la hauteur (16) de la plaque à os dans la région de la vis à os (12) est inférieure ou égale au diamètre (19) de la tige (18), **en ce que** la tête (13) de la vis à os (12) est réalisée sous forme de cuvette sphérique, dont la surface extérieure (22) et la surface intérieure (23) présentent un même centre (24), **en ce que** la vis de blocage (15) plonge dans la cuvette avec un noyau adapté (26) de sorte que, dans l'état non encore fixé, la vis à os (12) est capable de basculer, et **en ce que**, au moins dans quelques positions en basculement de la vis à os (12), la tête de vis (13) plonge dans un évidement (20) de la vis de blocage (15).

2. Agencement de liaison selon la revendication 1, **caractérisé en ce que** la vis de blocage (15) présente une longueur filetée (27) plongeant dans la plaque à os (7) qui s'élève à plus de la moitié de la hauteur (16) de la plaque à os (7) dans la région de la vis à os (12).

3. Agencement de liaison selon la revendication 1 ou 2, **caractérisé en ce que** l'évidement (20) de la vis de blocage (15) est choisi de telles dimensions que la tête (13) de la vis à os (12) peut être fixée sous des positions angulaires différentes par rapport à l'axe de la vis de blocage (15).

4. Agencement de liaison selon la revendication 3, **caractérisé en ce que**, en se référant à une position moyenne de la vis à os (12) dans la direction de l'axe de la vis de blocage (15), la tête (13) permet au niveau de sa surface extérieure (22) une position de fixation avec une déviation angulaire α1, et **en ce que** l'évidement (20) de la vis de blocage (15) permet pour la tête de vis (13) une déviation angulaire α2 d'amplitude analogue, et **en ce qu'**un angle β couvert par la surface antagoniste (10, 25) jusqu'au point médian est supérieur à l'angle respectif α1, α2.

5. Agencement de liaison selon la revendication 4, **caractérisé en ce que** les angles α1, α2 correspondent respectivement à un angle de 3° à 20°.

6. Agencement de liaison selon l'une des revendications 1 à 5, comprenant une plaque à os (7) qui est réalisée à la manière d'un étrier, lequel est utilisable à titre de corps d'ancrage (4, 14) pour une construction de soutien sur une vertèbre (1, 2).

7. Agencement de liaison selon l'une des revendications 1 à 5, comprenant une plaque à os (7) qui est utilisable à titre de pont (21) entre deux vertèbres (1, 2).

8. Agencement de liaison selon la revendication 7, comprenant un pont (21) qui comble la distance entre deux vertèbres (1, 2) et qui est réalisé à la manière d'un ressort de flexion (35).

9. Agencement de liaison selon l'une des revendications 1 à 8, **caractérisé en ce que** le diamètre (19) de la tige de la vis à os (12) est entre 2 et 10 mm.
